# EUROPEAN PATENT APPLICATION

(11) **EP 0 744 618 A2**
(43) Date of publication of application: **27.11.1996**
(21) Application number: 96660015.7
(22) Date of filing: 24.05.1996
(51) Int. Cl.: G01N 27/60, G01N 33/38

(54) **A method for the control of milling and mixing processes in the limestone, cement and mineral material industry**

(30) Priority: 26.05.1995 FI 952561
(71) Applicant: NORDKALK OY AB, 21600 Parainen (FI)
(72) Inventor: Rosin, Tomas, 20540 Turku (FI); Lindeberg, Tom, 08100 Lojo (FI); Nilsdorff, Stig, 21600 Pargas (FI); Jansson, Harry, 21600 Pargas (FI); Dahlberg, Kjell, 21600 Pargas (FI); Vihma, Maija, 20540 Abo (FI)
(74) Representative: Blomquist, Tuula Maria Ingeborg

(57) **Abstract**

The present invention refers to a method for monitoring and for control of milling and mixing processes in the limestone, cement and mineral material industry. The static electricity of a material flow of a supplied particle suspension or a product particle suspension is according to the invention continuously detected with a measuring system comprising a sensor means, a detector, a voltage meter and a processing unit. The detector includes a capacitive circuit connected between the sensor means and the voltage meter, the detector thereby has an integrating effect on the measurement signals read by the voltage meter. These measurement signals are transformed in the processing unit for continuous monitoring and control of the milling or mixing process.

## Description

The present invention relates to a method for monitoring and/or for the control of milling and mixing processes in the limestone, cement and mineral material industry.

Up to now reliable and rapid systems have not been available for on-line monitoring and control of the conditions in milling and mixing processes in the limestone and cement industry. It has been generally difficult to make measurements on the product in the difficult, dusty and abrasive conditions prevailing in e.g. a limestone or cement product mill. Generally samples have been taken from the production and analyzed outside the process in order to control the process. The sampling, and the analysis of the sample, is time consuming. Thus a process control based on these samples is realized with a relatively long delay.

The object of the present invention is to provide a method in which the above mentioned disadvantages are minimized.

Thus the object of the present invention particularly is to provide a method for continuous monitoring and control of milling and mixing processes in the limestone, cement and mineral material industry.

The above aims are attained by a method which is characterized in what is presented in the enclosed claims.

According to the invention the static electricity in a material flow of a supplied particle suspension and/or product suspension is continuously detected with a measuring system comprising a sensor means, a detector, a voltage meter, and a processing unit. The detector contains a capacitive circuit connected between the sensor means and the voltage meter, the capacitive circuit having an integrating effect on the measurement signals read by the voltage meter. From these measurement signals the processing unit calculates parameters for a continuous monitoring and/or control of the milling or mixing process.

The invention is described in more detail below with reference to the following figures, in which:
figure 1 shows schematically an embodiment of the measuring system according to the invention; and
figure 2 shows a measurement signal detected by a measurement system according to figure 1.

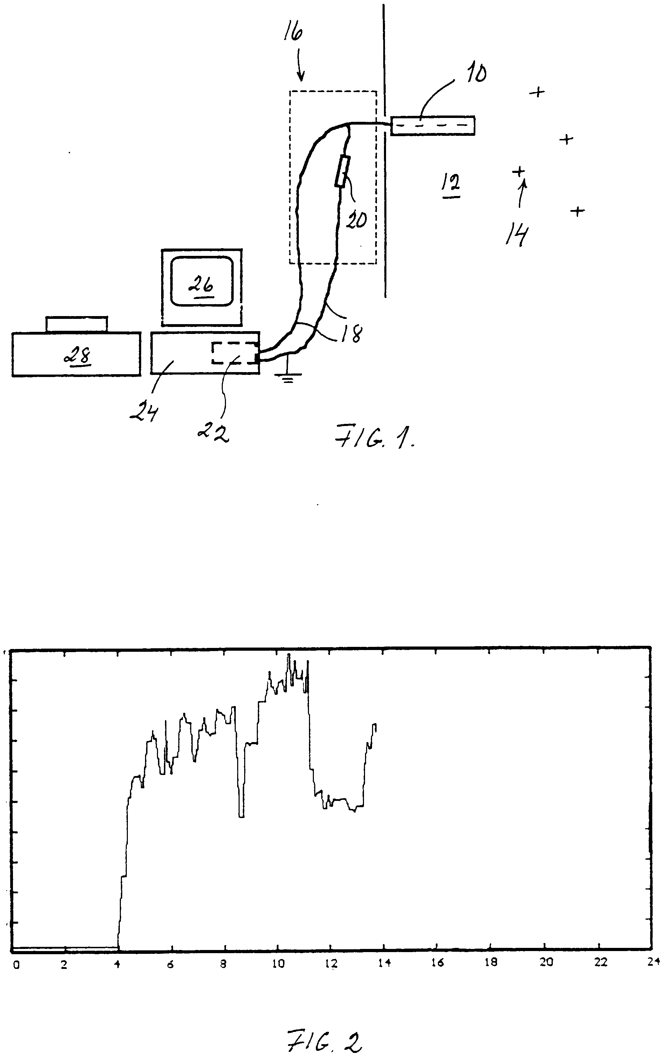

Figure 1 shows a measurement system according to the invention in which a sensor means, a so called antenna 10, is inserted in a tube 12 adjacent a sample flow 14 of charged particulate material which is to be milled or mixed, or which has been milled or mixed. The antenna is connected to a capacitive circuit 16 containing a cable 18 and a resistor 20. The capacitive circuit is connected to a voltage meter 22 in a processing unit 24. A monitor 26 and a printer 28 are connected to the processing unit. The antenna is shown on a larger scale than the other parts of the system.

The static electricity of a sample flow in the input duct and/or in the output duct of a milling or mixing device is simply detected with an antenna inserted in the duct. The antenna may be formed by e.g. a metal electrode or a simple conducting metal screw, which is inserted in the corresponding duct through an opening in the duct wall 30, as shown in figure 1.

The capacitive circuit is realized by a circuit connected between the antenna and the voltage meter, the circuit comprising a capacitor, or a signal cable 18 as is shown in figure 1, having a high capacitance C > 1 nF and a resistor having a high resistance > 1 Mohm.

A measurement system according to the invention can be utilized in many ways, e.g. to control the supply of different basic materials or additives in processes, to control the reaction time in processes, to control the degree of milling of basic materials or products, to control heat supply in processes, and/or to control combustion processes. The charge ratios between basic materials and products provides a possibility to see the effects of different control parameters on the processes.

The milling process is a typical process where a measurement of the electrical field of the particles can be used for process control. During milling a triboelectric charge is generated in the dielectric material, in other words a so called friction charge of the particles is generated when they contact each other and/or other material particles. The charges occur on the material surface, whereby the specific surface of the material is of a vital importance for the charging of the material.

The static electricity of the particles acts with a force on other charged particles reaching their vicinity. When particles with opposite polarities come close to each other a force interaction is generated between them. If the attractive force is sufficiently high, then the particles can be drawn together and form agglomerations. In a corresponding way a force interaction is generated between surfaces and adjacent particles having opposite polarities. If the interactive force is sufficiently high it causes deposits on the surfaces.

Agglomerations and deposits on the surfaces easily cause disturbances in the milling process. During milling there develops clean surfaces which can become strongly charged. If the particle surfaces are charged with the opposite polarity it results in particle agglomeration. Agglomerations an deposits of the surfaces can be prevented by using milling agents. A milling agent is often a polar substance in liquid form. The electric field from a positively charged particle turns the molecules of the milling agent so that the negative pole is pulled towards the field. In a corresponding way the positive pole of the milling agent is pulled towards a negatively charged particle. The milling agent is drawn to the particle surface and thereby it prevents other particles to be attached to the particle, in other words it prevents e.g. agglomerations or deposits on the surfaces. A milling agent represents an extra cost, which must be kept as low as possible.

Particle agglomerations produce weaker electrical fields than corresponding particles with an addition of a milling agent, as charges of opposite polarities cancel each other. When the electrical field is measured according to this invention it is thus possible to get an idea about the agglomeration degree of the material and/or to get an idea of the need for a milling agent. The measurement system can be used to control the addition of milling agents.

On the other hand, as the specific surface of particles is a quadratic function of the diameter, i.e. the smaller the particle diameter the larger the specific surface and thus the higher the specific charge per kilogram of mass, then a measurement of the electric field can also give an idea about the milling degree. The measurement system according to the invention can be utilised to control the residence time in the mill, for example.

An electrically charged powder generates a so called static electric "pillow", along which the particles slide without a too strong mutual contact. Thus static electricity can impart good flowing properties for certain powders. The stronger the electric field generated by the powder particles the better flowing properties can be expected of the powder. Thus a measurement of the electric field also provides an indication of the flowing properties of the powder.

The measurement system according to the invention is well suited for on-line control of different milling processes in the limestone, cement and mineral material industry. The object of the milling is to keep a uniform powder quality with a certain particle size distribution without agglomerations. The particle size depends on several different variables, such as the material flow, the length of stay in the mill, the temperature, the milling agent concentration, the negative pressure in the mill, separation steps, etc. These variables have a strong linkage, which previously complicated the control of the milling and made it difficult to obtain a product specification with narrow variations. However, with the measurement system according to the invention the effect of each variable on the product, i.e. on the electric field of the product, can be seen ea sily and rapidly. The effect of a desired variable can be controlled by varying the frequency of the standard deviation. Thus the measurement system provides a possibility to optimize the milling process. For instance, even if one of the variables is changed it is possible to retain an optimal process by compensating for it with another variable.

Coal, which is milled before the combustion in a limestone or cement product kiln, must be dried before the milling. The moisture content, the particle size and particularly the amount of agglomerations in the supplied coal spray, i.e. in the suspension of coal, air and steam, are very important regarding the combustion efficiency. The charge balance in the spray has probably also a certain effect on the combustion. The charge balance is directly dependent on the charges in the coal spray. With the measurement system according to the invention it is possible to control the milling and the preparation of the coal spray so that the generation of agglomerates is prevented, or at least substantially decreased, and so that the charges in the spray are kept on a suitable level.

Thus the method according to the invention is preferably used:
- to control or monitor milling or mixing of limestone based products, such as calcium oxide, hydrated lime, limestone, dolomite, cement or slag;
- to control or monitor coal milling before the coal is supplied to a lime or cement kiln; and
- to control or monitor milling or mixing of a mix of minerals, such as quartz, feldspar, talc, wollastonite or kaoline.

In a limestone mill the mass flow of limestone and milling agents and the negative pressure in the mill comprise the main parameters which influence the particle size. It was found that these parameters have an effect on the particle charge after the mill. It is then also possible to get an idea of the magnitude of these parameters by examining the particle charges according to the present invention.

The method according to the invention can be used to monitor simultaneously the particle suspensions at different points of the process, in order to control, on the basis of these results, different parameters in the process and different characteristics of the product, such as:
- the fineness and uniformity of the product;
- the flowing characteristics with or without milling agent;
- the slaking degree of quick-lime; or
- the humidity in a milling or mixing process.

Then the method can also be used for the optimization of chemical additive dosage, such as the addition of milling agent in milling or mixing processes. In experiments we found a strong correlation between the milling agent addition and the measurement signals obtained by the present new method. It is assumed that the electrical characteristics of both the product particles and the milling agent have an effect on the measurement signals.

Figure 2 shows a range of measurement signals detected by a measurement system according to figure 1. The antenna of the measurement system was inserted in the sample flow after a limestone mill. The concentration of the milling agent in the product can be clearly read from the measurement signals. The vertical axis indicates the signal magnitude and the horizontal axis indicates the time. The mill was started at moment 4, and then the additive level was kept at a desired level until moment 8. At that level a non-agglomerating product was obtained having suitable flowing characteristics. At the moment 8 there was a silo change, which also can be clearly read from the signal. Thereafter, during the period from 9 to 11, the additive level was too high. In other words, unnecessary much of the possibly expensive chemical was added. A correction was made only during the period from 11 to 13, to a too low level, the material thereafter possibly having a tendency to agglomerate. The additive level was corrected so that at moment 13 it was optimal. Thus, by examining the signals it is easy to control the additive level towards a suitable level. This is an essential improvement in the milling process.

We have also been able to observe a correlation between the mass flow of the limestone supplied to the mill and the measurement signals, and between the negative pressure in the mill and the measurement signals. A good idea about how the different parameters affect the measurement signal is obtained when the electrostatic charge is continuously detected in the input or output mass flows, and thereby it is also possible to interpret those signals which are obtained by the detection. On the basis of the measurement signals it is for instance easy to have a rapid detection of different process disturbances and to follow the course of events during the milling. Previously a disturbance could prevail a relatively long time before detection.

The invention shall not be limited to the applications presented above, but it can be widely applied within the inventive idea, which is defined in the claims presented below.

## Claims

1. A method for monitoring and/or for the control of milling and mixing processes in the limestone, cement and mineral material industry, **characterized** in that
- the static electricity of a material flow of a supplied particle suspension and/or product particle suspension is continuously detected with a measuring system comprising a sensor means, a detector, a voltage meter and a processing unit, whereby the detector contains a capacitive circuit connected between the sensor means and the voltage meter and whereby the detector has an integrating effect on the measurement signals read by the voltage meter, and that
- these measurement signals are transformed in the processing unit for continuous monitoring and/or control of the milling or mixing process.

2. A method according to claim 1, **characterized** in that the static electricity is read by a sensor means, a so called antenna, which is inserted in an input duct and/or an output duct of a milling or mixing device.

3. A method according to claim 1, **characterized** in that the capacitive circuit is realized by a circuit connected between the sensor means and the voltage meter, whereby the circuit comprises a signal cable or a capacitor having a capacitance C > 1 nF and a resistor R > 1 Mohm.

4. A method according to claim 1, **characterized** in that the method is used to control or monitor the milling or mixing of products based on limestone, such as calcium oxide, hydrated lime, limestone, dolomite, cement or slag.

5. A method according to claim 1, **characterized** in that the method is used to control or monitor the milling of coal before it is supplied to a limestone or cement product kiln.

6. A method according to claim 1, **characterized** in that the method is used to control or monitor the milling or mixing of a mix of minerals, such as quartz, feldspar, wollastonite, talc or kaoline.

7. A method according to claim 1, **characterized** in that the method is used to control or monitor the fineness, the uniformity, the flowing characteristics with or without a milling agent, the slaking degree, or the humidity of the product in a milling or mixing process.

8. A method according to claim 1, **characterized** in that the method is used to optimize the chemical addition dosage in milling or mixing processes.
